# EUROPÄISCHE PATENTSCHRIFT

(11) **EP 1 686 860 B1**
(45) Veröffentlichungstag und Bekanntmachung des Hinweises auf die Patenterteilung: **14.01.2009**
(21) Anmeldenummer: 04818811.4
(22) Anmeldetag: 17.11.2004
(51) Int. Cl.: A01N 59/14

(54) **ANTIBAKTERIELLES ADDITIV**
ANTI-BACTERIAL ADDITIVE
ADDITIF ANTIBACTERIEN

(30) Priorität: 18.11.2003 DE 10354245
(43) Veröffentlichungstag der Anmeldung: 09.08.2006
(73) Patentinhaber: AMI Agrolinz Melamine International GmbH, 4021 Linz (AT)
(72) Erfinder: JAVOR, Tatjana, A-4209 Engerwitzdorf (AT); SCHWESIG, Arthur, A-6850 Dornbirn (AT)
(74) Vertreter: Gross, Felix
(86) Internationale Anmeldenummer: PCT/EP2004/013219
(87) Internationale Veröffentlichungsnummer: WO 2005/048716

(56) Entgegenhaltungen:
- EP-A- 0 009 006
- EP-A- 1 205 439
- WO-A-01/76366
- WO-A-03/009827
- DATABASE WPI Section Ch, Week 199711 Derwent Publications Ltd., London, GB; Class A97, AN 1997-113947 XP002321443 & JP 09 001508 A (RENTOKIL LTD) 7. Januar 1997 (1997-01-07)
- DATABASE WPI Section Ch, Week 198350 Derwent Publications Ltd., London, GB; Class C03, AN 1983-841250 XP002321444 & JP 58 189104 A (KAO CORP) 4. November 1983 (1983-11-04)
- DATABASE WPI Section Ch, Week 198921 Derwent Publications Ltd., London, GB; Class A87, AN 1989-153902 XP002321445 & JP 01 093506 A (KATO Y) 12. April 1989 (1989-04-12)
- DATABASE WPI Section Ch, Week 199608 Derwent Publications Ltd., London, GB; Class A21, AN 1996-073680 XP002321446 & JP 07 329265 A (NITTO BOSEKI CO LTD) 19. Dezember 1995 (1995-12-19) in der Anmeldung erwähnt

## Beschreibung

Die Erfindung betrifft ein antibakterielles Additiv für Melaminharze gemäß Anspruch 1, ein antibakterielles Melaminharz gemäß Anspruch 11, ein Verfahren zur Herstellung eines solchen Melaminharzes gemäß Anspruch 19, ein antibakterielles Laminat gemäß Anspruch 23, ein Verfahren zur Herstellung eines antibakteriellen Laminates gemäß Anspruch 24, sowie die Verwendung eines antibakteriellen Laminates gemäß Anspruch 26.

Melaminharze finden vielfache industrielle Anwendung beispielsweise für die Beschichtung von Oberflächen oder auch für die Herstellung von dekorativen Laminaten. Unter den Melaminharzen haben Melamin - Formaldehyd - und Melamin / Harnstoff - Formaldehydharze die größte technische Bedeutung. Durch ihre ausgezeichneten Eigenschaften wie Kratzfestigkeit, Flammfestigkeit, chemische und mechanische Beständigkeit sowie ihre Härte sind sie überaus geeignet für stark beanspruchte Gegenstände und vor allem Oberflächen des täglichen Gebrauchs. Klassische Anwendungen von Melaminharzen sind beispielsweise in Form von Melaminharzlaminaten für Fußböden oder für Möbeloberflächen.

Aufgrund steigenden weltweiten Bedarfs solcher Laminate und Einsatz auch in Bereichen, in denen hygienisch kritische Bedingungen herrschen, besteht Nachfrage an Oberflächen mit antibakteriellen Eigenschaften. Antibakteriell bedeutet, dass die Gesamtkeimanzahl auf der betreffenden Oberfläche über einen gewissen Zeitraum lang konstant oder sogar abnehmend ist. Speziell in dichtbevölkerten Regionen mit bedenklichen epidemologischen Rahmenbedingungen im Wohnbereich und im öffentlichen Bereich schaffen antibakterielle Oberflächen die Grundlage für ein Anheben der Lebensqualität und der Gesundheit der Bevölkerung. Darüber hinaus bieten beispielsweise im Bereich von Krankenhäusern und Biolabors antibakterielle Oberflächen zusätzliche Sicherheit vor Kontaminationen.

Aus der JP 61258079 A sind antibakterielle Polyesterfasern bekannt, welche eine antibakterielle Wirksubstanz sowie Alkylenglykol enthaltende Acrylkomponenten oder Melaminkomponenten enthalten und eine dauerhafte antibakterielle Wirkung aufweisen.

Aus der Literatur sind weiters Additive bekannt, die. Melaminharze antibakteriell ausstatten.

Die JP 08073702 A betrifft beispielsweise antibakterielle Melaminharze, die eine Mischung von Aluminium-, Magnesium- und Siliziumoxiden sowie elementares Silber und Zink als antibakteriellen Wirkstoff enthalten. Damit können antibakterielle Eigenschaften für bis zu 48 Stunden erreicht werden. Der Nachteil dieses Additives ist seine kurz andauernde antibakterielle Eigenschaft und die große Anzahl an antibakteriellen Einzelkomponenten; je mehr Einzelkomponenten einem Melaminharz zugegeben werden, umso schwieriger ist es, alle Bestandteile der Harzflotte mengenmäßig optimal aufeinander abzustimmen.

In der JP 07329265 A wird ebenfalls ein antibakterielles Melaminharz in Form eines dekorativen Panels beschrieben. Ein Overlay Papier wird mit einem antibakteriellen Melaminharz imprägniert und anschließend mit einem oder mehreren phenolharzgetränkten Kempapieren zu einem Laminat verpresst. Das Melaminharz erhält seine antibakteriellen Eigenschaften durch eine der Komponenten Silber, Benzalkonium, Cetylpyridinium oder Isopropylmethylphenol. Um die antibakterielle Komponente kompatibel mit dem Melaminharz zu machen, muss sie in einem separaten Verarbeitungsschritt auf ein laminares Phosphat als Trägermaterial aufgebracht werden. Erst in dieser Form kann sie dem Melaminharz zugegeben werden.

Die WO 03/009827 A1 betrifft Melaminharze, die antimikrobiale Substanzen enthalten. Die antimikrobiale Komponente ist dabei eine Mischung aus einem Diphenyletherderivat und Orthophenylphenol, einer Substanz mit unerwünscht hohem Dampfdruck, deren Einsatz nur in speziellen Fällen gerechtfertigt ist. Nachteilig an dieser Mischung ist, dass es leicht von der Harzoberfläche ausgewaschen werden kann, so dass die antibakterielle Wirkung mit der Zeit abnimmt.

Als weitere antibakterielle Komponenten sind beispielsweise Zink- oder Natriumpyrithione, Azole, Hydrochlorid, Carbanilid sowie Silber, Kupfer und Zink in Zeolit oder amorphem Glaspulver beschrieben. Da einige dieser Komponenten mit dem Melaminharz reagieren können, müssen sie vor dem Zusatz zum Melaminharz in ein mit dem Melaminharz kompatibles Trägermaterial eingekapselt werden, was einen zusätzlichen hohen Arbeitsaufwand bedeutet.

Die US 6248342 B1 beschreibt antibakterielle Laminate, in deren melaminharzgetränkte Oberfläche eine anorganische, ein Metallion enthaltende antibakterielle Wirksubstanz eingearbeitet ist. Als antibakterielle Wirksubstanz werden bevorzugt Zeolite verwendet, welche Metallionen wie beispielsweise Ag, Cu, Zn, Hg, Sn, Pb, Bi, Cd, Cr oder Mischungen davon enthalten. Durch lonenaustauschprozesse gelangen die Metallionen an die Laminatoberfläche und ermöglichen so die antibakterielle Wirkung. Nachteilig dabei ist, dass die Metallionen während des lonenaustauschprozesses in deren Oxide, Hydroxide oder sonstige Salze umgewandelt werden können, sich in dieser Form an der Laminatoberfläche absetzen und somit dort die antibakterielle Wirkung abschwächen. Ein weiterer Nachteil aus gesundheitlicher Sicht ist der Einsatz von Schwermetallen bei Verwendung dieser Zeolite.

Die Verwendung von Zinkborat als flammhemmendes Mittel ist u.a. aus JP 67363/1981, JP 137988/1988 und der EP 1205439 A1 bekannt. Letztere beschreibt ein kristallines Zinkborat gemäß der Formel ZnO*mB₂O₃ *XH₂O mit m = 2,8 bis 3,2 und X kleiner gleich 4 mit einer Partikelgröße von 40 nm und mit einem sehr geringen Anteil an Natrium. Das Salz verteilt sich sehr gut in Harzen, verfügt über gute Flammenschutz- und rauchunterdrückende Eigenschaften und wirkt antibakteriell. Dieses speziell hergestellte Zinkborat ist jedoch in seiner Herstellung sehr kostenintensiv.

Es stellte sich demnach die Aufgabe, ein antibakterielles Additiv für Melaminharze zu entwickeln, welches obengenannte Nachteile nicht aufweist. Neben einer effektiven und dauerhaften antibakteriellen Wirkung werden an ein solches Additiv weitere Anforderungen gestellt. Es soll möglichst keine gesundheitlich bedenklichen Substanzen enthalten und es soll die charakteristischen Material-und Verarbeitungseigenschaften wie Imprägnier- und Härtungsverhalten des Melaminharzes nicht negativ beeinflussen. Weiter soll das Additiv in möglichst einfacher Art und Weise in das Melaminharz eingearbeitet werden können. Darüber hinaus soll es mit dem Harz kompatibel sein und dauerhaft in das Melaminharz-eingebunden sein.

Gegenstand der vorliegenden Erfindung ist daher ein antibakterielles Additiv für Melaminharze, insbesondere für Melamin / Formaldehyd oder Melamin / Harnstoff / Formaldehyd Harze, mit mindestens einem Boratsalz aus der Gruppe der Salze der Orthoborsäure H₃BO₃ und/oder der Metaborsäure HBO₂ und/oder von Polyborsäuren Hₙ₋₂BₙO₂ₙ₋₁ als antibakteriellen Wirkstoff und mindestens einer Verbindung die eine quaternäre Ammoniumverbindung der Formel 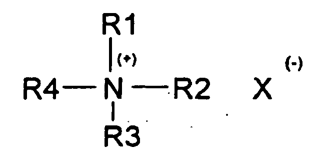 mit R₁ R₂, R₃ = C₁-C₅-Alkyl, R₄ = C₁-C₂₀ Akyl oder Benzyl, wobei R₁, R₂, R₃ und R₄ gleich oder verschieden sein können und X = Chlorid oder Bromid ist, oder Benzalkoniumchlorid ist, wobei das Additiv mindestens ein Boratsalz der Formel ZnₐB_{b}O_{c} * dH₂O mit a = 1 oder 2, b = 1 bis 8; c = 1 bis 13 und d = 0 bis 10 aufweist.

Ein Vorteil des erfindungsgemäßen antibakteriellen Additivs ist, dass durch die Verwendung von Boratsalzen die Anzahl und die Menge der im Additiv enthaltenen Wirkstoffe gering gehalten werden kann. Auf diese Weise ist die quantitative Abstimmung mit den sonstigen Bestandteilen der Melaminharzflotte unproblematisch.

Ein weiterer Vorteil eines antibakteriellen Additivs mit mindestens einem Boratsalz der genannten Formel und mindestens einer quaternären Ammoniumverbindung ist die synergistische Wirkung der antibakteriellen Wirkstoffe. Dies bedeutet, dass für das Erzielen einer bestimmten antibakteriellen Wirksamkeit bei Verwendung einer Mischung eine geringere Gesamtmenge als bei Verwendung der Einzelkomponenten nötig ist. Des Weiteren sind die eingesetzten Wirkstoffe des Additivs gesundheitlich unbedenklich.

Darüber hinaus sind sie ohne weitere zusätzliche Arbeitsschritte wie beispielsweise Einkapseln in oder Aufbringen auf ein Trägermaterial mit dem Melaminharz kompatibel. Die Wirkstoffe werden während des ermöglichen somit eine dauerhaft gleichbleibende antibakterielle Eigenschaft der Laminate.

Das erfindungsgemäße Additiv wird insbesondere für Melamin-Formaldehyd- oder Harnstoff/Melamin-Formaldehydharze verwendet.

Weitere vorteilhafte Beispiele für verwendbare Melaminharze sind solche, die durch Kondensation von Melamin oder Mischungen von Harnstoff mit Melamin mit Aldehyden der Kettenlängen C₁-C₁₀ entstehen. Auch Mischungen von Aldehyden dieser Kettenlängen, wie beispielsweise Formaldehyd, Acetaldehyd, Trimethylolacetaldehyd, Acrolein, Benzaldehyd, Furfural, Glyoxal, Glutaraldehyd, Phthalaldehyd, Terephthalaldehyd, Isobutyraldehyd, Aceton oder Ketone, wie beispielsweise Methylethylketon und Diethylketon sind möglich.

Beispiele für Melaminharze sind auch solche, die durch Umsetzung mit C₁-C₄-Alkoholen verethert und anschließend gegebenenfalls mit C₄-C₁₈- Alkoholen umgeethert werden. Solche Melaminharzes sind beispielsweise in der WO 03/046053 A1 beschrieben.

Weiters sind solche Melaminharzen möglich, die nach der Veretherung beispielsweise mit Diolen partiell umgeethert und / oder mit Bisepoxiden partiell umgesetzt werden.

Der Vorteil solcher modifizierter Melaminharze ist, dass sie nach thermoplastischen Verarbeitungsmethoden wie beispielsweise Extrusion oder Spritzguss verarbeitet werden können. Mit dem erfindungsgemäßen antibakteriellen Additiv können auf diese Weise Formteile mit antibakteriellen Eigenschaften hergestellt werden.

Die Melaminharze können Füllstoffe und Modifikatoren wie beispielsweise Elastifikatoren sowie Härter, Netzmittel, Trennmittel oder sonstige übliche Zusätze enthalten.

Die Boratsalze der Orthoborsäure H₃BO₃, der Metaborsäure HBO₂ oder von Polyborsäuren Hₙ₋₂ Bₙ O₂ₙ₋₁ werden mangels eines einheitlichen Nomenklatursystems üblicherweise über empirische Formeln beschrieben, beispielsweise über die Anzahl der Kationen und Bor-Atome in der einfachsten stöchiometrischen Einheit. Auch Oxidformeln oder die Namen der entsprechenden Borat-Minerale werden als Beschreibungsmerkmale verwendet.

Bevorzugterweise werden im erfindungsgemäßen Additiv weitere Boratsalze verwendet, die durch folgende Formeln beschrieben werden können:
Mₐ B_{b} O_{c} * d H₂O und/oder
Mₐ Nₐ B_{b}O_{c}* d H₂O wobei
a, a'= 1 oder 2
b = 1 bis 8
c = 1 bis 13
d = 0 bis10
M, N = NH₄, Na, K, Li, Ca, Mg, und wobei
M, N, a und a' gleich oder verschieden sein können.

Mögliche weitere Borate im erfindungsgemäßen Additiv sind beispielsweise Na₂B₄O₇*dH2O mit d = 0,5 oder 10; NaBO₂*dH₂O mit d = 2 oder 4; NaB₅O₈*5H₂O; Na₂B₈O₁₃*4H₂O; Ca₂B₆O₁₁*5H₂O; NaCaB₅O₉*dH₂O mit d = 5 oder 8; LiBO₂*8 H₂O; LiB₅O₈*5H₂O; Li₂B₄O₇*3H₂O; K₂B₄O₇*4H₂O; KB₅O₈*4H₂O; NH₄B₅O₈*4H₂O oder, (NH₄)₂B₄O*4H₂O;

In einer besonders bevorzugten Ausführungsform des erfindungsgemäßen antibakteriellen Additivs ist mindestens ein Boratsalz technisches Zinkborat ZuO * B₂O₃ * dH₂O und technisches Natriumborat Na₂O * B₂O₃ *10 H₂O, wobei das technische Zinkborat ≥ 45 Gew% ZnO und ≥ 36 Gew% B₂O₃ aufweist.

Besonders vorteilhaft ist weiteres ein antibakterielles Additiv, welches als einziges Boratsalz technisches Zinkborat ZnO * B₂O₃ * d H₂O aufweist.

Besonders vorteilhaft ist weiteres ein antibakterielles Additiv, welches als einziges Boratsalz technisches Zinkborat ZnO * B₂O₃ * d H₂O aufweist.

Dabei ist es vorteilhaft, wenn die Menge an Boratsalz im Additiv 0,1 bis 3 Gew%, bevorzugt 1 bis 2,5 Gew%, besonders bevorzugt 1,8 bis 2,2 Gew%, bezogen auf die Menge des festen Melaminharzes beträgt.

Der Vorteil dieser Boratsalze als antibakterielle Wirkstoffe ist, dass mit ihnen besonders gute antibakterielle Eigenschaften im Melaminharz erzielt werden können. Ein weiterer Vorteil dabei ist, dass die dafür benötigte Wirkstoffmenge gering ist. Eine geringe Wirkstoffmenge ist deshalb positiv, weil das Additiv dann technisch leicht handhabbar ist, das heißt, gut mit dem Melaminharz vermischt werden kann.

In einer besonders vorteilhaften Ausführungsform wird als quaternäre Ammoniumverbindung Benzalkoniumchlorid verwendet. Als Benzalkoniumchlorid wird die Substanz Alkylbenzyldimethylammoniumchlorid bezeichnet, wobei R im Verkaufsprodukt nicht einheitlich definiert ist. 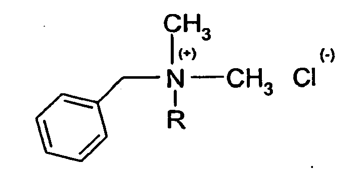 Benzalkoniumchlorid ist eine auf dem Markt erhältliche Substanz, die sowohl in flüssiger als auch in fester Form angeboten wird. Somit kann je nach den technischen Gegebenheiten wie beispielsweise Viskosität des verwendeten Melaminharzes die optimale Einsatzform gewählt werden.

Besonders bevorzugt ist ein antibakterielles Additiv, das eine Mischung aus technischem Zinkborat ZnO * B₂O₃ * dH₂O und technischem Natriumborat Na₂O * B₂O₃ * dH₂O mit d = 10 und Benzalkoniumchlorid aufweist, wobei das Gewichtsverhältnis technisches Zinkborat und technisches Natriumborat: Benzalkoniumchlorid im Melaminharz vorteilhafterweise 2 : 2 : 1 ist.

Weiterhin bevorzugt ist es, wenn die Menge an technischem Zinkborat ZnO * B₂O₃ * 3,5 H₂O und technischem Natriumborat Na₂O * B₂O₃ * 10 H₂O und die Menge an Benzalkoniumchlorid im Additiv 0,1 bis 1 Gew%, bevorzugt 0,2 bis 0,6 Gew%, bezogen auf die Menge des festen Melaminharzes, ist.

Mit einem solchen Additiv ist eine sehr gute antibakterielle Wirksamkeit bei gleichzeitig sehr guter technischer Verarbeitbarkeit während der Laminatherstellung gegeben. Beim Imprägnieren eines Flächengebildes mit einem das Additiv enthaltenden antibakteriellen Melaminharz ist entscheidend, dass sich die im Melaminharz enthaltenen antibakteriellen Wirkstoffe des Additivs gleichmäßig auf dem Flächengebilde verteilen. Dies wird bei einer Ausführungsform, in der das Melaminharz eine Mischung von Zinkborat und / oder Natriumborat und Benzalkoniumchlorid enthält, besonders gut erreicht.

Ein weiterer Gegenstand der vorliegenden Erfindung ist ein antibakterielles Melaminharz, das ein erfindungsgemäßes antibakterielles Additiv enthält sowie ein Verfahren zur Herstellung eines solchen antibakteriellen Melaminharzes.

Bei der Herstellung des erfindungsgemäßen antibakteriellen Melaminharzes wird ein in gelöster Form vorliegendes Melaminharz mit üblichen Mischvorrichtungen wie beispielsweise Rührern mit dem erfindungsgemäßen antibakteriellen Additiv vermischt. Dabei ist es wichtig, dass die Mischung aus antibakteriellem Additiv und Melaminharz gut gerührt wird, sodass eine möglichst gleichmäßige antibakterielle Melaminharzsuspension erhalten wird, in welcher die Teilchen andauernd in Schwebe gehalten werden.

Üblicherweise liegt das Melaminharz, dem das Additiv zugemischt wird, als wässrige oder alkoholische Lösung vor.

Das Additiv kann dem Melaminharz in fester und/ oder flüssiger Form zugemischt werden. Es ist möglich, die einzelnen Additivbestandteile individuell oder als Mischung der Bestandteile zuzugeben. Das im Additiv enthaltene Boratsalz kann gemeinsam mit und/oder nach und/oder vor der gegebenenfalls enthaltenen quaternären Ammoniumverbindung dem Melaminharz zugemischt werden. Nach der Additivzugabe kann gegebenenfalls der Härter als letzte Komponente dem Melaminharz zugegeben werden.

Es wird ein antibakterielles Melaminharz in suspendierter Form.erhalten, welches anschließend direkt, beispielsweise für die Laminatherstellung, weiterverarbeitet oder, beispielsweise durch Sprühtrocknen, in die Form eines Festharzes überführt und zu einem späteren Zeitpunkt seiner weiteren Verarbeitung zugeführt werden kann.

Bevorzugt ist eine Ausführungsform, bei welcher das antibakterielle Additiv während der Melaminharzsynthese zugemischt wird. In diesem Fall wird das bei der Melaminharzsynthese erhaltene Melaminharzvorkondensat abgekühlt und anschließend mit dem Additiv vermischt. Vorteilhaft dabei ist, dass direkt aus den Rohstoffen für die Harzsynthese ohne zusätzlichen Zwischenisolierungsschritt des Melaminharzes ein antibakterielles Melaminharz hergestellt werden kann.

Weiter bevorzugt ist eine Ausführungsform, bei welcher das antibakterielle Additiv dem Melaminharz nach der Melaminharzsynthese zugemischt wird. In diesem Fall kann das Melaminharz als Flüssigharz in gelöster Form oder als Festharz vorliegen. Liegt es als Festharz vor, wird es vor dem Vermischen mit dem Additiv in die gelöste Form überführt.
Bei dieser Ausführungsform ist vorteilhaft, dass jedes beliebige auf dem Markt erhältliche Melaminharz durch einfaches Vermischen mit dem erfindungsgemäßen Additiv in ein antibakterielles Melaminharz überführt werden kann.

Ein weiterer Gegenstand der vorliegenden Erfindung ist ein antibakterielles Laminat enthaltend ein antibakterielles Melaminharz sowie ein Verfahren zur Herstellung eines solchen antibakteriellen Laminates.

Für die Laminatherstellung wird mindestens ein trockenes saugfähiges Flächengebilde mit dem antibakteriellen Melaminharz imprägniert.

Das saugfähige Flächengebilde liegt beispielsweise in Form von Papier, Pappe, Gewebe oder Vlies, Holzfurnieren, Holzfaserplatten oder Holzspanplatten vor, es enthält bevorzugt Cellulose und / oder Lignocellulose.

Das für die Laminatherstellung verwendete antibakterielle Melaminharz kann als Festharz oder als Flüssigharz vorliegen, üblicherweise wird es in Form einer wässrigen Lösung zur Laminatherstellung eingesetzt. Es kann weitere Zusatzstoffe wie beispielsweise Netz- oder Trennmittel, Plastifikatoren und Härter sowie sonstige übliche Zusätze enthalten.

Der Auftrag an antibakteriellem Melaminharz, bezogen auf das ursprünglich eingesetzte Flächengebilde beträgt üblicherweise 110 bis 130 Gew%.

Dabei wird ein antibakterielles Flächengebilde erhalten, welches vor der weiteren Verarbeitung getrocknet wird.

Das getrocknete antibakterielle .Flächengebilde wird anschließend mit mindestens einem harzimprägnierten Flächengebilde als Zwischenschicht oder mit einem Trägermaterial wie beispielsweise Pressspanplatten bei üblichen Druck- und Temperaturbedingungen zu einem Laminat verpresst und dabei vollständig ausgehärtet.

Das so erhaltene antibakterielle Laminat weist neben den für Melaminharz-Laminate typischen exzellenten Werkstoff- und Oberflächeneigenschaften ausgezeichnete dauerhafte antibakterielle Eigenschaften auf.

Durch die antibakterielle Wirksamkeit der erfindungsgemäßen Melaminharze gegen ein sehr breites Bakterienspektrum und sehr aggressive Bakterienkulturen bieten sich vielfältige Anwendungsmöglichkeiten. Beispielsweise können aus thermoplastisch verarbeitbaren Melaminharzen antibakterielle Formteile hergestellt werden. Weiterhin können antibakterielle Pressmassen hergestellt werden. Darüber hinaus bieten die aus den Harzen hergestellten antibakteriellen Laminate vielfältige Anwendungsmöglichkeiten. So können sie für Möbeloberflächen oder Fußböden in allen Bereichen eingesetzt werden, wo besonderer Wert auf hygienische Bedingungen gelegt wird wie beispielsweise in Bad, Küche oder auch in Krankenhäusern.

### Beispiel 1:

### Herstellung des antibakteriellen Melamin Formaldehyd Harzes

500 g Melamin/Formaldehyd Harz (Agrolinz Melamin Italia) werden in 500 g Wasser gelöst, anschließend werden 3 g des Netzmittels Melpan NU02MF und 5 g des Härters Melpan A462 (herkömmliche Netz- und Trennmittel der Firma Agrolinz Melamin Italia) zugefügt. Diese wässrige Aminoplastvorkondensat-Mischung wird gerührt, bis eine klare Lösung erhalten wird.

Zu dieser Melaminharzlösung werden anschließend
- bei der Probe D 10 g technisches Zinkborat und
- bei der Probe E 2,5 g technisches Zinkborat, 2,5 g technisches Natriumborat und 1,25 g Benzalkoniumchlorid zugemischt
und 10 min gerührt bis eine homogene Suspension entsteht.

### Herstellung des antibakteriellen Laminates

Mit dieser Suspension des antibakteriellen Melaminharzes wird ein weisses Dekorpapier (Dichte 80 g/m²) imprägniert. Das imprägnierte Dekorpapier wird anschließend 1 Stunde an der Luft, dann für 90 sec lang bei 120°C im Trockenschrank bis zu einer Restfeuchte von etwa 7-8 Gew% getrocknet. Diese einmalige Imprägnierung ergab einen Gesamtharzauftrag von ca. 120-130 Gew% bezogen auf die Masse des ursprünglich eingesetzten trockenen weißen Dekorpapiers.
Dieses mit antibakteriellem Melamin/Formaldehyd Harz imprägnierte Dekorpapier wird mit vier Lagen eines mit einem Melamin/Formaldehyd Harz imprägnierten Kraftkernpapiers und eines ebenfalls mit einem Melamin/Formaldehyd Harz imprägnierten Gegenzugpapiers zu einem Mehrschichtlaminat verpresst. Dabei werden folgende Pressbedingungen angewandt: Pressdauer 2 min, Presstemperatur 150°C, Pressdruck 80 kg/cm², Rückkühlung auf 70°C.
Das so erhaltene antibakterielle Laminat weist eine glänzende und transparente Oberfläche auf.

### Untersuchung der antibakteriellen Wirkung

Als Maß für die antibakterielle Wirkung wird die Resistenz von Gram positiven und Gram negativen Bakterien untersucht. Die antimikrobielle Aktivität vom antibakteriellen Melamin/Formaldehyd Harz über einen Zeitraum von 24 Stunden wurde zunächst an dreizehn Bakterienarten und einer Hefe getestet. Die Bakterienarten waren *Escherichia coli* 14*, Klebsiella pneumoniae* 13, *Proteus vulgaris* 14*, Salmonella typhi* 1*, Staphylococcus aureus* 5*, Enterococcus faecalis* 1*, Streptococcus pyogenes* A22, *Pseudomonas aeruginosa, Staphylococcus saprophyticus, Staphylococcus haemoliticus, Pseudomonas fluorescens, Staphylococcus epidermis, Vibrio parahaemoliticus.* Die Hefe war *Candida albicans* 494-Art.
Die in dieser antimikrobischen Untersuchung verwendeten Bakterienarten waren aus menschlichen Infektionen isoliert worden.

Zur Untersuchung der antimikrobiellen Aktivität wurde die Folienkontaktmethode in der modifizierten Form verwendet. Jede Probe wurde in einer trypischen Sojabrühe gezüchtet, um eine Mikrobenzahl von 10⁹ koloniebildenden Einheiten (colony-forming units CFUs) pro ml zu erreichen. Anschließend wurden diese Kulturen mit sterilen Phosphatpuffern verdünnt, um Testkulturen von etwa 10⁵ CFUs/ml zu gewinnen. Vergleichbare Mengen der verdünnten Brühenkulturen wurden anschließend auf die folgenden Proben aufgegeben:
1) Nährstoffagar in einer Petrischale (Kontrollmuster)
2) Standard Melaminharz Probe A (enthält kein antibakterielles Additiv)
3) Antibakterielles Melamin/Formaldehyd Harz Probe D
4) Antibakterielles Melamin/Formaldehyd Harz Probe E
   Nach 0, 4, 8 und 24 Stunden wurden die Proben der aufgetragenen Kulturen entfernt, nacheinander im Verhältnis 1:10, 1:100 und 1:1000 verdünnt und anschließend auf Mueller Hinton Agar (DIFCO) und Sabouraud-Dextrose-Agar (nur bei Candida albicans) platziert, um die Reduktion der lebensfähigen Bakterien als CFUs zu bestimmen.

### Ergebnisse

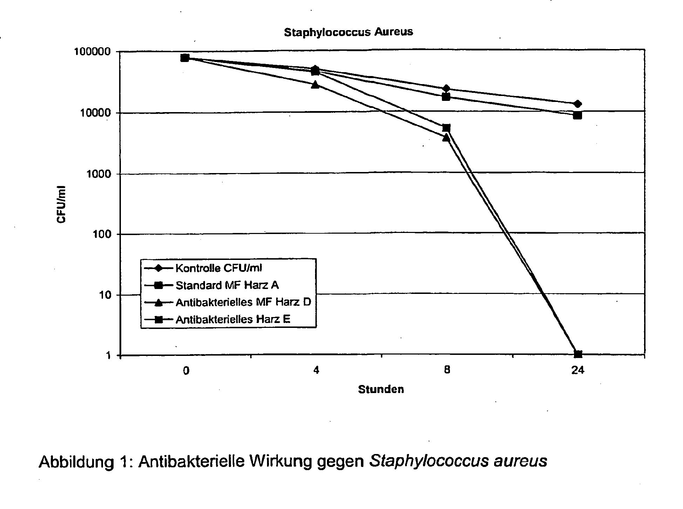

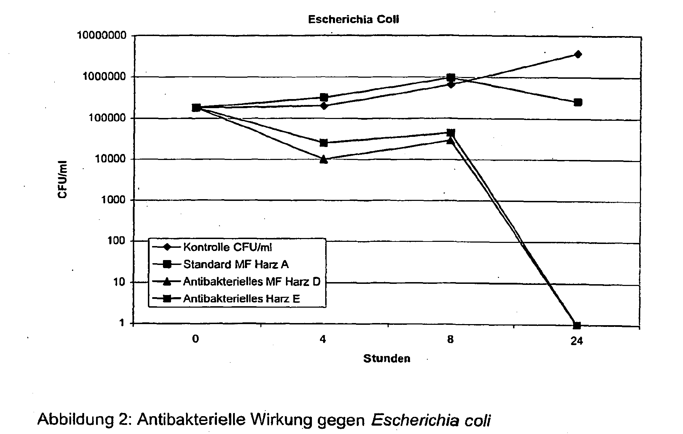

Die Ergebnisse der antibakteriellen Wirkung der additivierten
Melamin/Formaldehyd Harze für Bakterien *Staphylococcus aureus* und *Escherichia coli* sind in den Abbildungen 1 und 2 dargestellt. Es sind die Proben D und E dargestellt, wobei die Probe D Zinkborat und die Probe E Zinkborat, Natriumborat und Benzalkoniumchlorid als antibakterielle Wirkstoffe enthält.

Aus den Abbildungen 1 und 2 ist ersichtlich, dass bei Verwendung der erfindungsgemäßen Borate als antibakterielle Additive (Proben D und E) eine deutlich raschere Abnahme der Bakterienkonzentration im Melamin/Formaldehyd Harz auftritt als dies im nicht-additivierten Melamin/Formaldehyd Harz (Standard MF Harz A) der Fall ist.
Dies gilt wie aus den Abbildungen ersichtlich ist, für verschiedene Bakterienarten wie beispielsweise *Staphylococcus aureus* und *Escherichia coli.*

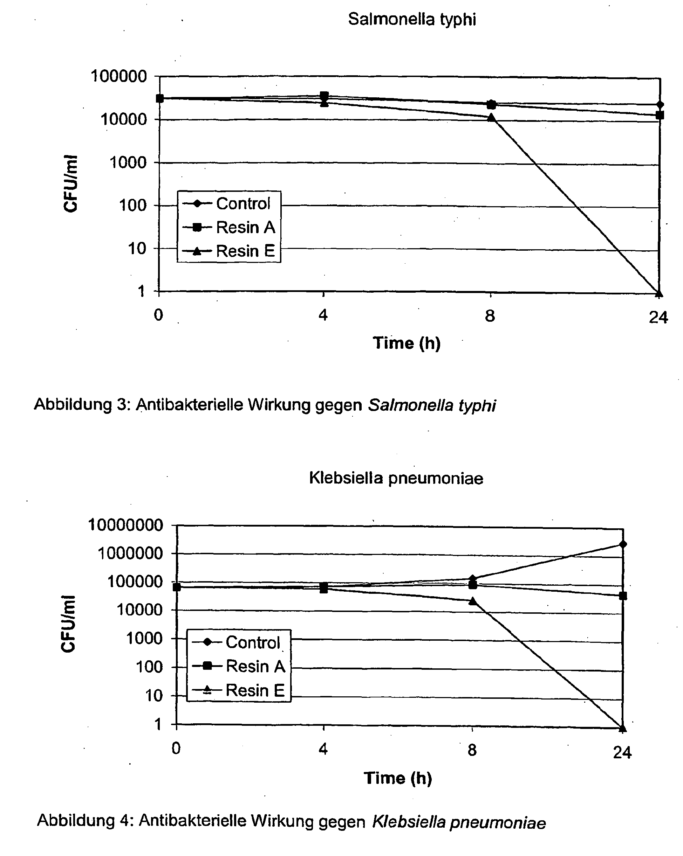

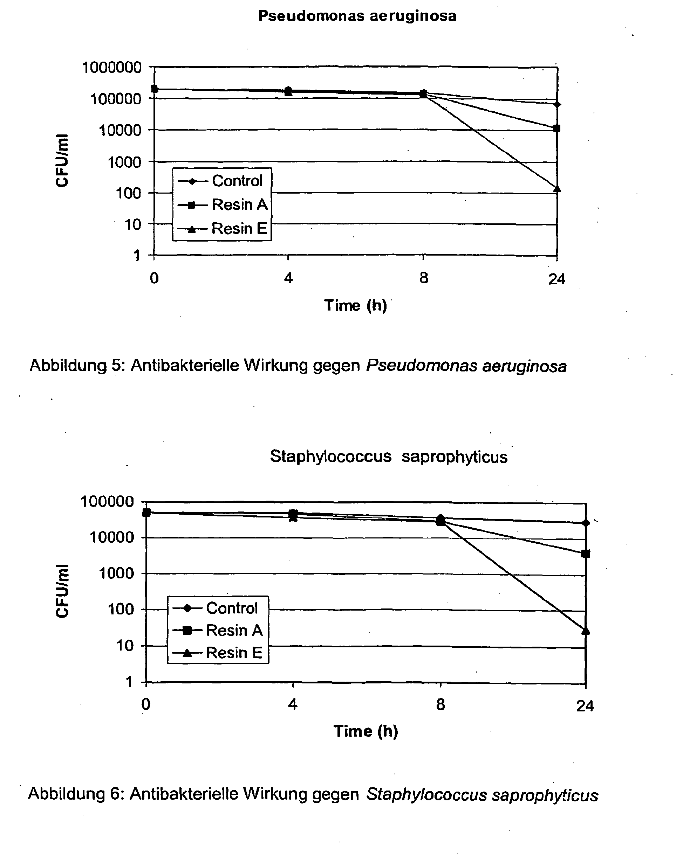

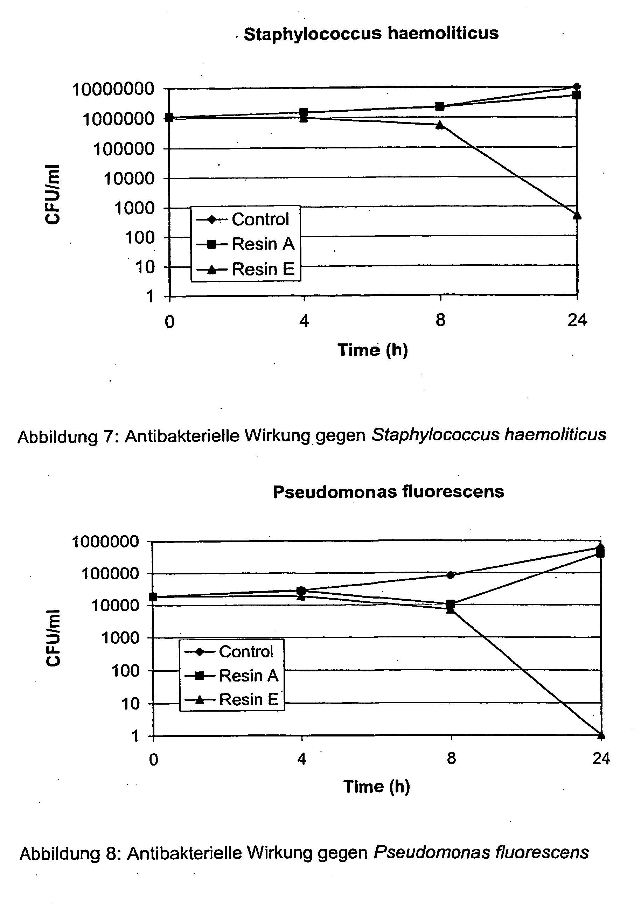

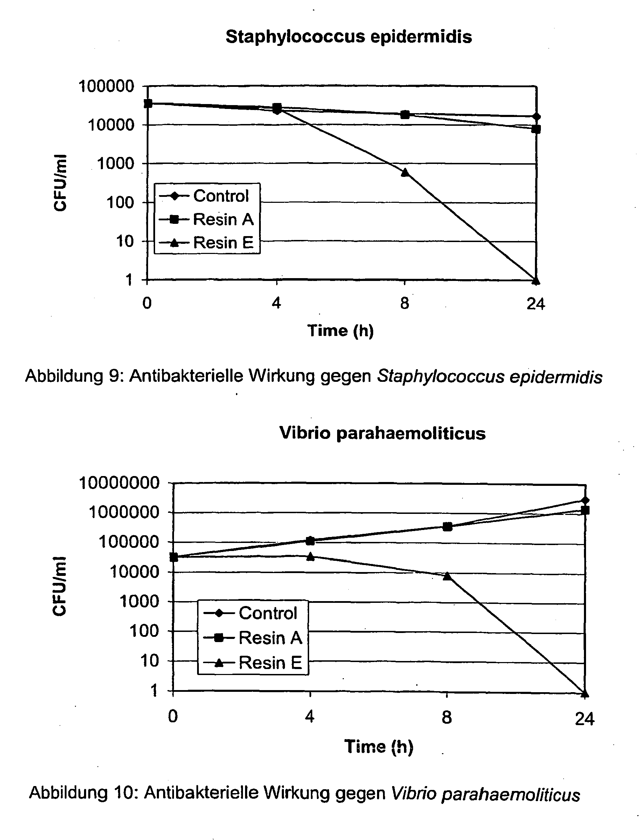

In den Abbildungen 3 bis 10 ist die antibakterielle Wirkung der Additive in Probe E gegen unterschiedlichste Bakterienarten dargestellt.
Die Probe E wurde nach derselben Methode, wie in Beispiel 1. unter "Herstellung des antibakteriellen Melamin Formaldehyd Harzes" beschrieben, hergestellt. Der einzige Unterschied bestand darin, dass der Härter als letzte Komponente, das heißt nach der Zugabe des Additivs, dem Melaminharz zugefügt wird.
Es ist ersichtlich, dass das erfindungsgemäße Additiv bei allen Bakterienarten zu einer raschen Abnahme der Bakterienkonzentration gegenüber einem nicht mit dem Additiv behandelten Melamin/Formaldehyd Harz führt.

## Patentansprüche

1. Antibakterielles Additiv für Melaminharze mit mindestens einem Boratsalz aus der Gruppe der Salze der Orthoborsäure H₃BO₃ und/oder der Metaborsäure HBO₂ und/oder von Polyborsäuren Hₙ₋₂BₙO₂ₙ₋₁ als antibakteriellen Wirkstoff und mindestens einer Verbindung, die eine quaternäre Ammoniumverbindung der Formel 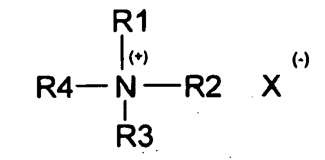 mit R₁, R₂, R₃ = C₁-C₅-Alkyl, R₄ = C₁-C₂₀ Akyl oder Benzyl, wobei R₁, R₂, R₃ und R₄ gleich oder verschieden sein können und X = Chlorid oder Bromid ist, oder Benzalkoniumchlorid ist,
**gekennzeichnet durch**
mindestens ein Boratsalz der Formel ZnₐBbO_{c}* dH₂O mit a = 1 oder 2, b = 1 bis 8; c = 1 bis 13 und d = 0 bis 10.

2. Antibakterielles Additiv für Melaminharze nach Anspruch 1 für Melamin-Formaldehyd oder Melamin/Harnstoff-Formaldehydharze.

3. Antibakterielles Additiv für Melaminharze nach Anspruch 1 oder 2, **dadurch gekennzeichnet, dass** die Melaminharze durch Kondensation von Melamin oder Mischungen von Harnstoff mit Melamin mit Aldehyden oder Mischungen von Aldehyden wie beispielsweise Formaldehyd, Acetaldehyd, Trimethylolacetaldehyd, Acrolein, Benzaldehyd, Furfural, Glyoxal, Glutaraldehyd, Phthalaldehyd, Terephthalaldehyd, Isobutyraldehyd, Aceton oder Ketonen wie beispielsweise Methylethylketon und Diethylketon entstehen.

4. Antibakterielles Additiv für Melaminharze nach einem der vorher genannten Ansprüche, **dadurch gekennzeichnet, dass** die Melaminharze durch Umsetzung mit C₁-C₄-Alkoholen verethert und/oder verethert und anschließend mit C₄-C₁₈- Alkoholen und/oder Diolen umgeethert und/oder verethert und mit Bisepoxiden partiell umgesetzt werden.

5. Antibakterielles Additiv für Melaminharze nach einem der vorher genannten Ansprüche **gekennzeichnet durch** mindestens ein weiteres Boratsalz der Formel
Mₐ B_{b} O_{c} * d H₂O und/oder
Mₐ Nₐ B_{b} O_{c} * d H₂O wobei
a, a' = 1 oder 2
b = 1 bis 8
c = 1 bis 13
d = 0 bis 10
M, N = NH₄, Na, K, Li, Ca, Mg und wobei
M, N, a und a' gleich oder verschieden sein können.

6. Antibakterielles Additiv nach mindestens einem der vorher genannten Ansprüche, **dadurch gekennzeichnet, dass** mindestens ein weiteres Boratsalz Na₂B₄O₇ * dH2O mit d = 0, 5 oder 10; NaBO₂ *dH₂O mit d = 2 oder 4; NaB₅O₈*5H₂O; Na₂B₈O₁₃*4H₂O; Ca₂B₆O₁₁*5H₂O; NaCaB₅O₉*dH₂O mit d = 5 oder 8; LiBO₂*8 H₂O; LiB₅O₈*5H₂O; Li₂B₄O₇*3H₂O; K₂B₄O₇*4H₂O; KB₅O₈*4H₂O; NH₄B₅O₈*4H₂O und/oder (NH₄)₂B₄O₇*4H₂O ist.

7. Antibakterielles Additiv nach mindestens einem der vorher genannten Ansprüche, **dadurch gekennzeichnet, dass** mindestens ein Boratsalz technisches Zinkborat ZnO * B₂O₃ * d H₂O mit ≥ 45 Gew% ZnO und ≥ 36 Gew% B₂O₃ ist.

8. Antibakterielles Additiv nach mindestens einem der vorher genannten Ansprüche, **dadurch gekennzeichnet, dass** mindestens ein weiteres Boratsalz technisches Natriumborat Na₂O * B₂O₃ * 10 H₂O ist.

9. Antibakterielles Additiv nach mindestens einem der vorher genannten Ansprüche, **dadurch gekennzeichnet, dass** es als einziges Boratsalz technisches Zinkborat ZnO * B₂O₃ * d H₂O aufweist.

10. Antibakterielles Additiv nach Anspruch 9, **dadurch gekennzeichnet, dass** es technisches Zinkborat ZnO * B₂O₃ *d H₂O und technisches Natriumborat Na₂O * B₂O₃ * d H₂O mit d = 10 und Benzalkoniumchlorid im Gewichtsverhältnis 2 : 2 : 1 aufweist.

11. Antibakterielles Melaminharz enthaltend ein antibakterielles Additiv nach mindestens einem der Ansprüche 1 bis 10.

12. Antibakterielles Melaminharz enthaltend ein antibakterielles Additiv nach mindestens einem der Ansprüche 1 bis 10, **dadurch gekennzeichnet, dass** die Menge an Boratsalz 0,1 bis 3 Gew%, bezogen auf die Menge des festen Melaminharzes, ist.

13. Antibakterielles Melaminharz enthaltend ein antibakterielles Additiv nach mindestens einem der Ansprüche 1 bis 10, **dadurch gekennzeichnet, dass** die Menge an Boratsalz 1 bis 2, 5 Gew%, bezogen auf die Menge des festen Melaminharzes, ist.

14. Antibakterielles Melaminharz enthaltend ein antibakterielles Additiv nach mindestens einem der Ansprüche 1 bis 10, **dadurch gekennzeichnet, dass** die Menge an Boratsalz 1,8 bis 2,2 Gew%, bezogen auf die Menge des festen Melaminharzes, ist.

15. Antibakterielles Melaminharz enthaltend ein antibakterielles Additiv nach Anspruch 10, **dadurch gekennzeichnet, dass** die Menge an technischem Zinkborat und Benzalkoniumchlorid 0,1 bis 1 Gew%, bezogen auf die Menge des festen Melaminharzes, ist.

16. Antibakterielles Melaminharz enthaltend ein antibakterielles Additiv nach Anspruch 10, **dadurch gekennzeichnet, dass** die Menge an technischem Zinkborat und Benzalkoniumchlorid 0,2 bis 0,6 Gew%, bezogen auf die Menge des festen Melaminharzes, ist.

17. Antibakterielles Melaminharz enthaltend ein antibakterielles Additiv nach Anspruch 10, **dadurch gekennzeichnet, dass** die Menge an technischem Zinkborat und technischem Natriumborat und Benzalkoniumchlorid 0,1 bis 1 Gew%, bezogen auf die Menge des festen Melaminharzes, ist.

18. Antibakterielles Melaminharz enthaltend ein antibakterielles Additiv nach Anspruch 10, **dadurch gekennzeichnet, dass** die Menge an technischem Zinkborat und technischem Natriumborat und Benzalkoniumchlorid 0,2 bis 0,6 Gew%, bezogen auf die Menge des festen Melaminharzes, ist.

19. Verfahren zur Herstellung eines antibakteriellen Melaminharzes nach Anspruch 11,
**dadurch gekennzeichnet,**
**dass** ein antibakterielles Additiv nach einem der Ansprüche 1 bis 10 mit einem in gelöster Form vorliegenden Melaminharz vermischt wird, wobei das Additiv dem Melaminharz in fester und/oder flüssiger Form zugemischt wird und wobei ein antibakterielles Melaminharz in suspendierter Form erhalten wird, welches anschließend direkt oder nach Überführung in ein Festharz zu einem späteren Zeitpunkt weiterverarbeitet wird.

20. Verfahren nach Anspruch 19, **dadurch gekennzeichnet, dass** das antibakterielle Additiv während der Melaminharzsynthese nach Abkühlen des bei der Melaminharzsynthese erhaltenen Melaminharzvorkondensates zugemischt wird.

21. Verfahren nach Anspruch 19, **dadurch gekennzeichnet, dass** das antibakterielle Additiv nach der Melaminharzsynthese zugemischt wird, wobei das Zumischen zu einem als Flüssigharz in gelöster Form vorliegenden Melaminharz erfolgt oder bei Vorliegen eines Festharzes das Zumischen nach Überführung des Festharzes in die gelöste Form erfolgt.

22. Verfahren nach einem der Ansprüche 19 bis 21, **dadurch gekennzeichnet, dass** das im Additiv enthaltene Boratsalz gemeinsam mit und/oder nach und/oder vor der quaternären Ammoniumverbindung mit dem Melaminharz vermischt wird.

23. Antibakterielles Laminat enthaltend ein antibakterielles Melaminharz nach Anspruch 11.

24. Verfahren zur Herstellung eines antibakteriellen Laminates nach Anspruch 23, **dadurch gekennzeichnet, dass**
a. ein trockenes saugfähiges Flächengebilde mit dem in gelöster Form vorliegenden antibakteriellen Melaminharz imprägniert wird,
b. das so erhaltene antibakterielle Flächengebilde getrocknet wird und
c. das getrocknete antibakterielle Flächengebilde mit einer oder mehrerer harzimprägnierter Zwischenschichten oder einem Trägermaterial zu einem Laminat verpresst und vollständig ausgehärtet wird.

25. Verfahren nach Anspruch 24, **dadurch gekennzeichnet, dass** das Melaminharz weitere Zusatzstoffe wie beispielsweise Netz- oder Trennmittel, Plastifikatoren und Härter sowie sonstige übliche Zusätze enthält.

26. Verwendung eines antibakteriellen Laminates nach Anspruch 23 für Oberflächen und Fußböden.

## Claims

1. Antibacterial additive for melamine resins with at least one borate salt from the group of the salts of orthoboric acid H₃BO₃ and/or of metaboric acid HBO₂ and/or of polyboric acids Hₙ₋₂BₙO₂ₙ₋₁ as active antibacterial compound and at least one compound, which is a quaternary ammonium compound of the formula 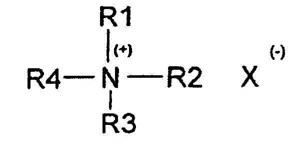 with R₁, R₂, R₃ = C₁-C₅ alkyl, R₄ = C₁-C₂₀ alkyl or benzyl, it being possible for R₁, R₂, R₃ and R₄ to be identical or different, and X = chloride or bromide, or benzalkonium chloride
**characterized by**
at least one borate salt of the formula ZnₐB_{b}O_{c} * dH₂O with a = 1 or 2, b = 1 to 8; c = 1 to 13 and d = 0 to 10.

2. Antibacterial additive for melamine resins according to Claim 1, for melamine-formaldehyde or melamine/urea-formaldehyde resins.

3. Antibacterial additive for melamine resins according to Claim 1 or 2, **characterized in that** the melamine resins are formed by condensation of melamine or of mixtures of urea with melamine with aldehydes or mixtures of aldehydes such as, for example, formaldehyde, acetaldehyde, trimethylolacetaldehyde, acrolein, benzaldehyde, furfural, glyoxal, glutaraldehyde, phthalaldehyde, terephthalaldehyde, isobutyraldehyde, acetone or ketones such as, for example, methyl ethyl ketone and diethyl ketone.

4. Antibacterial additive for melamine resins according to one of the preceding claims, **characterized in that** the melamine resins are etherified by reaction with C₁-C₄ alcohols and/or etherified and subsequently transetherified with C₄-C₁₈ alcohols and/or diols and/or etherified and partly reacted with bisepoxides.

5. Antibacterial additive for melamine resins according to one of the preceding claims, **characterized by** at least one further borate salt of the formula
Mₐ B_{b} O_{c}* d H₂O and/or
Mₐ Nₐ B_{b} O_{c} * d H₂O, where
a, a' = 1 or 2
b = 1 to 8
c = 1 to 13
d = 0 to 10
M, N = NH₄, Na, K, Li, Ca, Mg and where
M, N, a and a' may be identical or different.

6. Antibacterial additive according to at least one of the preceding claims, **characterized in that** at least one further borate salt is Na₂B₄O₇ * dH₂O where d = 0, 5 or 10; NaBO₂ *dH₂O where d = 2 or 4; NaB₅O₈*5H₂O; Na₂B₈O₁₃*4H₂O; Ca₂B₆O₁₁*5H₂O; NaCaB₅O₉*dH₂O where d = 5 or 8; LiBO₂*8 H₂O; LiB₅O₈*5H₂O; Li₂B₄O₇*3H₂O; K₂B₄O₇*4H₂O; KB₅O₈*4H₂O; NH₄B₅O₈*4H₂O and/or (NH₄)₂B₄O₇*4H₂O₇*4H₂O.

7. Antibacterial additive according to at least one of the preceding claims, **characterized in that** at least one borate salt is technical zinc borate ZnO * B₂O₃ * dH₂O withy 45% by weight ZnO and 36% by weight B₂O₃.

8. Antibacterial additive according to at least one of the preceding claims, **characterized in that** at least one further borate salt is technical sodium borate Na₂O * B₂O₃ * 10 H₂O.

9. Antibacterial additive according to at least one of the preceding claims, **characterized in that** as sole borate salt it has technical zinc borate ZnO * B₂O₃*dH₂O.

10. Antibacterial additive according to Claim 9, **characterized in that** it has technical zinc borate ZnO * B₂O₃ * dH₂O and technical sodium borate Na₂O * B₂O₃ * dH₂O with d = 10 and benzalkonium chloride in a weight ratio of 2:2:1.

11. Antibacterial melamine resin comprising an antibacterial additive according to at least one of Claims 1 to 10.

12. Antibacterial melamine resin comprising an antibacterial additive according to at least one of Claims 1 to 10, characterize in that the amount of borate salt is 0.1% to 3% by weight, based on the amount of solid melamine resin.

13. Antibacterial melamine resin comprising an antibacterial additive according to at least one of Claims 1 to 10, **characterized in that** the amount of borate salt is 1% to 2.5% by weight, based on the amount of solid melamine resin.

14. Antibacterial melamine resin comprising an antibacterial additive according to at least one of Claims 1 to 10, **characterized in that** the amount of borate salt is 1.8% to 2.2% by weight, based on the amount of solid melamine resin.

15. Antibacterial melamine resin comprising an antibacterial additive according to Claim 10, **characterized in that** the amount of technical zinc borate and benzalkonium chloride is 0.1% to 1% by weight, based on the amount of solid melamine resin.

16. Antibacterial melamine resin comprising an antibacterial additive according to Claim 10, **characterized in that** the amount of technical zinc borate and benzalkonium chloride is 0.2% to 0.6% by weight, based on the amount of solid melamine resin.

17. Antibacterial melamine resin comprising an antibacterial additive according to Claim 10, **characterized in that** the amount of technical zinc borate and technical sodium borate and benzalkonium chloride is 0.1% to 1% by weight, based on the amount of solid melamine resin.

18. Antibacterial melamine resin comprising an antibacterial additive according to Claim 10, **characterized in that** the amount of technical zinc borate and technical sodium borate and benzalkonium chloride is 0.2% to 0.6% by weight, based on the amount of solid melamine resin.

19. Process for producing an antibacterial melamine resin according to Claim 11, **characterized in that** an antibacterial additive according to any one of Claims 1 to 10 is mixed with a melamine resin present in dissolved form, the additive being admixed to the melamine resin in solid and/or liquid form to give an antibacterial melamine resin in suspended form which subsequently, directly or at a later point in time, following conversion into a solid resin, is processed further.

20. Process according to Claim 19, **characterized in that** the antibacterial additive is admixed during the melamine resin synthesis after the melamine resin precondensate obtained in the melamine resin synthesis has cooled.

21. Process according to Claim 19, **characterized in that** the antibacterial additive is admixed after the melamine resin synthesis, the admixing taking place to a melamine resin present in dissolved form as a liquid resin, or, where a solid resin is present, the admixing taking place after the solid resin has been converted into the dissolved form.

22. Process according to one of Claims 19 to 21, **characterized in that** the borate salt present in the additive is mixed with the melamine resin together with and/or after and/or before the quaternary ammonium compound.

23. Antibacterial laminate comprising an antibacterial melamine resin according to Claim 11.

24. Process for producing an antibacterial laminate according to Claim 23, **characterized in that**
a. a dry absorbent sheetlike structure is impregnated with the antibacterial melamine resin present in dissolved form,
b. the antibacterial sheetlike structure thus obtained is dried, and
c. the dried antibacterial sheetlike structure is pressed with one or more resin-impregnated interlayers or with a support material, to form a laminate, and is fully cured.

25. Process according to Claim 24, **characterized in that** the melamine resin comprises further additives such as, for example, wetting agents or release agents, plasticizers and curing agents and also other customary additions.

26. Use of an antibacterial laminate according to Claim 23 for surfaces and floors.

## Revendications

1. Additif antibactérien pour résines mélamine, comportant au moins un borate choisi dans le groupe des sels de l'acide orthoborique H₃BO₃ et/ou de l'acide métaborique HBO₂ et/ou d'acides polyboriques Hₙ₋₂BₙO₂ₙ₋₁, en tant substance active antibactérienne et au moins un composé qui est un composé d'ammonium quaternaire de formule 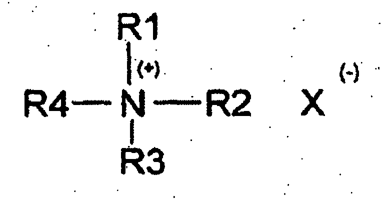 où R₁, R₂, R₃ = alkyle en C₁-C₅, R₄ =alkyle en C₁-C₂₀ ou benzyle, R₁, R₂, R₃ et R₄ pouvant être identiques ou différents et X = chlorure ou bromure, ou est le chlorure de benzalkonium,
**caractérisé par** au moins un borate de formule ZnₐB_{b}O_{c}.dH₂O, où a = 1 ou 2, b = 1 à 8 ; c = 1 à 13 et d = 0 à 10.

2. Additif antibactérien pour résines mélamine selon la revendication 1, pour résines mélamine-formaldéhyde ou mélamine/urée-formaldéhyde.

3. Additif antibactérien pour résines mélamine selon la revendication 1 ou 2, **caractérisé en ce que** les résines mélamine résultent de la condensation de mélamine ou de mélanges d'urée et de mélamine avec des aldéhydes ou des mélanges d'aldéhydes, comme par exemple le formaldéhyde, l'acétaldéhyde, le triméthylolacétaldéhyde, l'acroléine, le benzaldéhyde, le furfural, le glyoxal, le glutaraldéhyde, le phtalaldéhyde, le téréphtalaldéhyde, l'isobutyraldéhyde, l'acétone ou des cétones comme par exemple la méthyléthylcétone et la diéthylcétone.

4. Additif antibactérien pour résines mélamine selon l'une quelconque des revendications précédentes, **caractérisé en ce que** les résines mélamine sont éthérifiées par réaction avec des alcools en C₁-C₄ et/ou éthérifiées et ensuite trans-éthérifiées par des alcools en C₄-C₁₈ et/ou des diols et/ou éthérifiées et partiellement mises en réaction avec des bis-époxydes.

5. Additif antibactérien pour résines mélamine selon l'une quelconque des revendications précédentes, **caractérisé par** au moins un autre borate de formule
Mₐ B_{b} O_{c} * d H₂O et/ou de formule
Mₐ Nₐ B_{b} O_{c} * d H₂O, où
a, a' = 1 ou 2
b = 1 à 8
c = 1 à 13
d = 0 à 10
M, N = NH₄, Na, K, Li, Ca, Mg et où
M, N, a et a' peuvent être identiques ou différents.

6. Additif antibactérien selon au moins l'une des revendications précédentes, **caractérisé en ce que** ledit au moins un autre borate est Na₂B₄O₇*dH₂O où d = 0, 5 ou 10 ; NaBO₂*dH₂O, où d = 2 ou 4 ; NaB₅O₈*5H₂O ; Na₂B₈O₁₃*4H₂O ; Ca₂B₆O₁₁*5H₂O ; NaCaB₅O₉*dH₂O, où d = 5 ou 8 ; LiBO₂*8H₂O ; LiB₅O₈*5H₂O ; Li₂B₄O₇*3H₂O ; K₂B₄O₇*4H₂O ; KB₅O₈*4H₂O ; NH₄B₅O₈*4H₂O et/ou (NH₄)₂B₄O₇*4H₂O.

7. Additif antibactérien selon au moins l'une des revendications précédentes, **caractérisé en ce que** ledit au moins un borate est un borate de zinc industriel ZnO*B₂O₃*dH₂O comportant ≥ 45 % en poids de ZnO et ≥ 36 % en poids de B₂O₃.

8. Additif antibactérien selon au moins l'une des revendications précédentes, **caractérisé en ce que** ledit au moins un autre borate est un borate de sodium industriel Na₂O*B₂O₃*10H₂O.

9. Additif antibactérien selon au moins l'une des revendications précédentes, **caractérisé en ce qu'**il comporte comme unique borate du borate de zinc industriel ZnO*B₂O₃*dH₂O.

10. Additif antibactérien selon la revendication 9, **caractérisé en ce qu'**il comporte du borate de zinc industriel ZnO*B₂O₃*dH₂O et du borate de sodium industriel Na₂O*B₂O₃*dH₂O où d = 10 et du chlorure de benzalkonium en le rapport pondéral 2:2:1.

11. Résine mélamine antibactérienne contenant un additif antibactérien selon au moins l'une des revendications 1 à 10.

12. Résine mélamine antibactérienne contenant un additif antibactérien selon au moins l'une des revendications 1 à 10, **caractérisée en ce que** la quantité de borate va de 0,1 à 3 % en poids, par rapport à la quantité de la résine mélamine solide.

13. Résine mélamine antibactérienne contenant un additif antibactérien selon au moins l'une des revendications 1 à 10, **caractérisée en ce que** la quantité de borate va de 1 à 2,5 % en poids, par rapport à la quantité de la résine mélamine solide.

14. Résine mélamine antibactérienne contenant un additif antibactérien selon au moins l'une des revendications 1 à 10, **caractérisée en ce que** la quantité de borate va de 1,8 à 2,2 % en poids, par rapport à la quantité de la résine mélamine solide.

15. Résine mélamine antibactérienne contenant un additif antibactérien selon la revendication 10, **caractérisée en ce que** la quantité de borate de zinc industriel et de chlorure de benzalkonium va de 0,1 à 1 % en poids, par rapport à la quantité de la résine mélamine solide.

16. Résine mélamine antibactérienne contenant un additif antibactérien selon la revendication 10, **caractérisée en ce que** la quantité de borate de zinc industriel et de chlorure de benzalkonium va de 0,2 à 0,6 % en poids, par rapport à la quantité de la résine mélamine solide.

17. Résine mélamine antibactérienne contenant un additif antibactérien selon la revendication 10, **caractérisée en ce que** la quantité de borate de zinc industriel et de borate de sodium industriel et de chlorure de benzalkonium va de 0,1 à 1 % en poids, par rapport à la quantité de la résine mélamine solide.

18. Résine mélamine antibactérienne contenant un additif antibactérien selon la revendication 10, **caractérisée en ce que** la quantité de borate de zinc industriel et de borate de sodium industriel et de chlorure de benzalkonium va de 0,2 à 0,6 % en poids, par rapport à la quantité de la résine mélamine solide.

19. Procédé pour la préparation d'une résine mélamine antibactérienne selon la revendication 11, **caractérisé en ce qu'**on mélange un additif antibactérien selon l'une quelconque des revendications 1 à 10 avec une résine mélamine présente sous forme dissoute, en ajoutant l'additif sous forme solide et/ou sous forme liquide à la résine mélamine, pour obtenir une résine mélamine antibactérienne sous forme en suspension, qui est ensuite transformée directement ou après conversion en une réside solide à un moment ultérieur.

20. Procédé selon la revendication 19, **caractérisé en ce que** l'additif antibactérien est ajouté pendant la synthèse de la résine mélamine, après refroidissement du produit de précondensation de résine mélamine obtenu lors de la synthèse de la résine mélamine.

21. Procédé selon la revendication 19, **caractérisé en ce que** l'additif antibactérien est ajouté après la synthèse de la résine mélamine, l'addition s'effectuant à une résine mélamine en tant que résine liquide, présente sous forme dissoute ou, en présence d'une résine solide, l'addition s'effectuant après conversion de la résine solide en la forme dissoute.

22. Procédé selon l'une quelconque des revendications 19 à 21, **caractérisé en ce que** le borate contenu dans l'additif est mélangé avec la résine mélamine avec la résine mélamine conjointement avec et/ou après et/ou avant le composé d'ammonium quaternaire.

23. Stratifié antibactérien contenant une résine mélamine antibactérienne selon la revendication 11.

24. Procédé pour la fabrication d'un stratifié antibactérien selon la revendication 23, **caractérisé en ce que**
a. on imprègne un objet plat absorbant, sec, avec la résine mélamine antibactérienne présente sous forme dissoute,
b. on sèche l'objet plat antibactérien ainsi obtenu et
c. l'objet plat antibactérien séché est pressé en un stratifié avec une ou plusieurs couches intermédiaires imprégnées de résine ou un matériau de support, et totalement durci.

25. Procédé selon la revendication 24, **caractérisé en ce que** la résine mélamine contient d'autres additifs, comme par exemple des agents mouillant ou de séparation, des plastifiants et des durcisseurs ainsi que d'autres additifs usuels.

26. Utilisation d'un stratifié antibactérien selon la revendication 23 pour des surfaces et des sols.
